# EUROPÄISCHE PATENTANMELDUNG

(11) **EP 1 319 395 A2**
(43) Veröffentlichungstag der Anmeldung: **18.06.2003**
(21) Anmeldenummer: 02026707.6
(22) Anmeldetag: 30.11.2002
(51) Int. Cl.: A61K 7/42, A61K 9/10

(54) **Stabile wirkstoffhaltige Zubereitungen**

(30) Priorität: 17.12.2001 DE 10161884
(71) Anmelder: Beiersdorf AG, 20245 Hamburg (DE)
(72) Erfinder: Max, Heiner, Dr., 22529 Hamburg (DE); Hargens, Birgit, 20257 Hamburg (DE); Raschke, Thomas, Dr., 25421 Pinneberg (DE)

(57) **Zusammenfassung**

Kosmetische und/oder dermatologische Zubereitungen enthaltend mindestens einen wasserlöslichen Wirkstoff und mindestens einen partikulären organischen UV-Lichtschutzfilter.

## Beschreibung

Die vorliegende Erfindung betrifft wirkstoffhaltige kosmetische und/oder dermatologische Zubereitungen mit einem Gehalt an partikulären organischen Lichtschutzfiltern.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Aufgabe der kosmetischen Hautpflege ist es, die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) zu stärken oder wiederherzustellen.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Kosmetische und/oder dermatologische Zubereitungen zur Hautpflege enthalten in der Regel neben Feuchtigkeitsspendern wie Glycerin und die Haut fettende Substanzen wie Öle, Fette, Wachse, auch eine Reihe von Wirkstoffen, die beispielsweise einer vorzeitigen Hautalterung, insbesondere der Entstehung und Ausbreitung von Fältchen und Falten, entgegenwirken sollen. So werden vielfach Vitamine (Vitamin A, B, C, D, E, F, H) den Salben, Cremes und Lotionen zugesetzt. Auch α-Glucosylrutin, welches als Antioxidans wirkt, kann beispielsweise zur Prophylaxe der polymorphen Lichtdermatose eingesetzt werden. Diese Wirkstoffe sind meist gut wasserlöslich.

Derlei kosmetische und/oder dermatologische Zubereitungen müssen, insbesondere wenn sie in lichtdurchlässigen Gefäßen aufbewahrt werden, vor der Zersetzung durch Lichteinfluss geschützt werden. Dies kann einerseits durch die Verwendung lichtundurchlässiger Verpackungen geschehen oder aber durch Zusatz von UV-Lichtschutzfiltern, was den Vorteil hat, dass die Haut des Anwenders nach dem Auftragen der kosmetischen und/oder dermatologischen Zubereitung gleichzeitig vor den schädlichen Folgen des UV-Lichtes (z.B. des Sonnenlichts) geschützt wird.

Viele Anwender wirkstoffhaltiger Hautpflegeprodukte leiden an einer überempfindlichen und zu Allergien neigenden Haut. Sie streben nach Pflegeprodukten, deren Reizpotential für die Haut möglichst gering ist. Vielen wasser- und öllöslichen UV-Lichtschutzfiltern haftet der wenn auch in der Regel unbegründete Ruf an, die Haut zu reizen und Allergien zu provozieren. Da diese Lichtschutzfilter mit der kosmetischen Zubereitung in die Haut einziehen, werden die entsprechenden Produkte von den besorgten Verbrauchern häufig gemieden. Um auch dieser Zielgruppe wirkstoffhaltige Hautpflegeprodukte anbieten zu können, werden zunehmend unlösliche anorganische UV-Filterpigmente wie Titandioxid, Zinkoxid den Pflegeprodukten zugefügt, die weitgehend auf der Hautoberfläche liegen bleiben. Diese UV-Filterpigmente haben aber den großen Nachteil, dass sie nach relativ kurzer Zeit die Zubereitung gelblich-braun verfärben und zersetzen. Dieser Zersetzungsprozess tritt selbst bei Einsatz von oberflächenbeschichteten anorganischen Pigmenten auf.

Es war daher die Aufgabe der vorliegenden Erfindung stabile, wasserlösliche Wirkstoffe enthaltende kosmetische und/oder dermatologische Zubereitungen zu entwickeln, die unlösliche UV-Lichtschutzfilter enthalten und sich bei längerer Lagerung nicht verfärben.

Überraschend wird die Aufgabe gelöst durch kosmetische und/oder dermatologische Zubereitungen enthaltend mindestens einen wasserlöslichen Wirkstoff und mindestens einen partikulären organischen UV-Lichtschutzfilter. Diese Zubereitungen sind frei von unlöslichen anorganischen UV-Filterpigmenten wie Titandioxid oder Zinkoxid, wobei frei von unlöslichen anorganischen UV-Filterpigmenten erfindungsgemäß bedeutet, dass diese Substanzen in einer Menge von höchstens 0,05 Gewichts-% bezogen auf das Gesamtgewicht der Zubereitung enthalten sein können.

Die erfindungsgemäßen kosmetischen und/oder dermatologischen Zubereitungen enthalten die wasserlöslichen Wirkstoffe in einer Konzentration von 0,001 bis 10 Gewichts-%, bevorzugt in einer Konzentration von 0,01 bis 5 und ganz besonders bevorzugt in einer Konzentration von 0,1 bis 3 Gewichts-%, bezogen auf das Gesamtgewicht der Zubereitung.

Ausserdem enthalten die erfindungsgemäßen kosmetischen und/oder dermatologischen Zubereitungen die partikulären organischen Lichtschutzfilter in einer Konzentration von 0,01 bis 10 Gewichts-%, wobei der Konzentrationsbereich von 0,1 bis 5 Gewichts-% und insbesondere von 0,25 bis 3 Gewichts-%, jeweils bezogen auf das Gesamtgewicht der Zubereitung besonders bevorzugt ist.

Dabei ist es erfindungsgemäß vorteilhaft, wenn das Verhältnis von wasserlöslichen Wirkstoffen zu partikulären organischen Lichtschutzfiltern von 1 : 500 bis 50 : 1 und insbesondere von 1 : 250 bis 25: 1 und ganz besonders bevorzugt von 1 : 100 bis 10: 1 beträgt.

Als erfindungsgemäß bevorzugte wasserlösliche Wirkstoffe können Ascorbinsäure, α-Glucosylrutin, Carnitin sowie dessen wasserlösliche Derivate wie beispielsweise Acetylcarnitin, Grüntee-Flavonoide wie beispielsweise Epigallocatechingallat, Catechin oder Epigallocatechin, oder Creatin sowie dessen wasserlösliche Derivate eingesetzt werden, wobei der erfindungsgemäße Einsatz an Wirkstoffen selbstverständlich nicht auf die genannten Substanzen beschränkt sein soll. Der erfindungsgemäß besonders bevorzugte wasserlösliche Wirkstoff ist dabei Ascorbinsäure.

Als partikulärer organischer Lichtschutzfilter wird 2,2'-Methylen-bis-(6-(2H-benzotriazol-2-yl)-4-(1,1,3,3-tetramethylbutyl)-phenol) [Tinosorb M (Ciba)] erfindungsgemäß besonders bevorzugt eingesetzt.

Die erfindungsgemäßen kosmetischen und/oder dermatologischen Zubereitungen enthalten vorteilhafter Weise lineare, verzweigte und/oder cyclische Oligo- und/oder Polysaccharidderivate. Dabei ist es erfindungsgemäß besonders bevorzugt, die verzweigten und/oder cyclischen Oligo- und/oder Polysaccharidderivate aus der Gruppe der Cyclodextrine, nichtgelierenden Cellulosen, Natriumstärkeoctenylsuccinat, Distärkephosphat (CAS-Nr. 55 63-33-2) und Aluminiumstärkeoctenylsuccinat (CAS-Nr. 90 87-61-0) zu wählen. Unter Cyclodextrinen sind erfindungsgemäß zu verstehen die nativen Cyclodextrine α-, β- und γ-Cyclodextrin sowie die Derivate dieser Spezies, insbesondere alle ganz oder teilweise an den Hydroxylgruppen veretherten und/oder veresterten und/oder anders derivatisierten β- und γ-Cyclodextrine wie das Hydroxypropyl-ß-Cyclodextrin sowie das random-Methyl-β-Cyclodextrin (z.B. von der Firma Wacker-Chemie GmbH, Burghausen).

Erfindungsgemäß ganz besonders bevorzugte verzweigte und/oder cyclische Oligound/oder Polysaccharidderivate sind Hydroxypropyl-β-Cyclodextrin, γ-Cyclodextrine sowie Distärkephosphat.

Die linearen, verzweigten und/oder cyclischen Oligo- und/oder Polysaccharidderivate werden erfindungsgemäß bevorzugt in einer Konzentration von 0,001 bis 10 Gewichts-%, insbesondere von 0,1 bis 5 Gewichts-% und ganz besonders bevorzugt in einer Konzentration von 0,25 bis 3 Gewichts-% bezogen auf das Gesamtgewicht der Zubereitung eingesetzt.

Es ist dem Fachmanne natürlich bekannt, dass anspruchsvolle kosmetische Zusammensetzungen zumeist nicht ohne die üblichen Hilfs- und Zusatzstoffe denkbar sind. Die erfindungsgemäßen kosmetischen Zubereitungen können daher kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, die Haut selbstbräunende oder bleichende Mittel, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittelund Arzneimittelgesetz).

Die erfindungsgemäßen kosmetischen und/oder dermatologischen Zubereitungen können erfindungsgemäß vorteilhaft Antioxidantien und/oder Radikalfänger enthalten. Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Tocopherole und Derivate (z.B. Vitamin-E-acetat), sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.
Die Menge der vorgenannten Antioxidantien (eine oder mehrere Verbindungen) in den Zubereitungen beträgt vorzugsweise 0,001 bis 30 Gew.-%, besonders bevorzugt 0,01 - 20 Gew.-%, insbesondere 0,02 - 5 Gew.-%, bezogen auf das Gesamtgewicht der Zubereitung.
Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.
Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Es ist erfindungsgemäß bevorzugt, den beschriebenen Emulsionen Komplexbildner zuzufügen.
Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.
Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -lon zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.
Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen), Iminodibernsteinsäure und deren Anionen.
Der oder die Komplexbildner sind erfindungsgemäß vorteilhaft in kosmetischen oder dermatologischen Zubereitungen bevorzugt zu 0,001 Gew.-% bis 10 Gew.-%, bevorzugt zu 0,05 Gew.-% bis 5 Gew.-%, insbesondere bevorzugt zu 0,1 - 1,0 Gew.-%, bezogen auf das Gesamtgewicht der Zubereitungen, enthalten.
Zur Anwendung werden die kosmetischen und dermatologischen Zubereitungen erfindungsgemäß in der für Kosmetika üblichen Weise auf die Haut und/oder die Haare in ausreichender Menge aufgebracht.

Vorteilhaft können die erfindungsgemäßen Zubereitungen weitere Substanzen enthalten, die UV-Strahlung im UVA- und/oder UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Emulsionen mit mindestens einem UVB-Filter als Antioxidans bzw. die Verwendung einer Kombination der erfindungsgemäß verwendeten Wirkstoffkombinationen mit mindestens einem UVB-Filter als Antioxidans in einer kosmetischen oder dermatologischen Zubereitung.

Es kann auch erfindungsgemäß von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'isopropylphenyl)propan-1,3-dion.
Weiterhin vorteilhafte UVA-Filter entstammen der Gruppe der Triazine, so z.B. das 2,4-Bis-{[4-(2-Ethyl-hexyloxy)-2-hydroxy]-phenyl)-6-(4-methoxyphenyl)-1,3,5-triazin (Handelsbezeichnung Tinosorb® S) sowie der Gruppe der Triazole, wie z.B. das 2,2'-Methylen-bis- [6-2H-benzotriazol-2yl]-4-(1,1,3,3-tetramethylbutyl)phenol) (Handelsbezeichnung Tinosorb® M). Einen erfindungsgemäß vorteilhaften wasserlöslichen UVA-Filter stellt das 2'-bis-(1,4-Phenylen)-1H-benzimidazol-4,6-disulfonsäure-Natriumsalz dar (Handelsbezeichnung Neo Heliopan AP®).

Gegenstand der Erfindung ist auch die Verwendung einer Kombination von erfindungsgemäß verwendeten Emulsionen mit mindestens einem UVA-Filter als Antioxidans bzw. die Verwendung einer Kombination der erfindungsgemäßen Wirkstoffkombinationen mit mindestens einem UVA-Filter als Antioxidans in einer kosmetischen oder dermatologischen Zubereitung.

Gegenstand der Erfindung ist auch die Verwendung einer Kombination aus erfindungsgemäßen Emulsionen mit mindestens einem UVA-Filter und/oder mindestens einem UVB-Filter als Antioxidans in einer kosmetischen oder dermatologischen Zubereitung.

Erfindungsgemäße Zubereitungen können auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfatoder Sulfonatgruppen auf. In wäßriger Lösung bilden sie im sauren oder neutralen Milieu negativ geladene organische lonen. Kationische Tenside sind beinahe ausschließlich durch das Vorhandensein einer quaternären Ammoniumgruppe gekennzeichnet. In wäßriger Lösung bilden sie im sauren oder neutralen Milieu positiv geladene organische lonen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine lonen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate
Carbonsäuren und Derivate, wie
1. Carbonsäuren, beispielsweise Laurinsäure, Aluminiumstearat und Magnesiumalkanolat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 0,3 und 25 Gew.-% in den erfindungsgemäßen Zubereitungen vorliegen, bezogen auf das Gesamtgewicht der Zubereitungen.

Die Lipidphase der erfindungsgemäßen kosmetischen oder dermatologischen Emulsionen kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether, Dicaprylylcarbonat.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Solche Silicone oder Siliconöle können als Monomere vorliegen, welche in der Regel durch Strukturelemente charakterisiert sind, wie folgt:

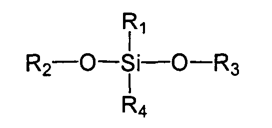

Als erfindungsgemäß vorteilhaft einzusetzenden linearen Silicone mit mehreren Siloxyleinheiten werden im allgemeinen durch Strukturelemente charakterisiert wie folgt: 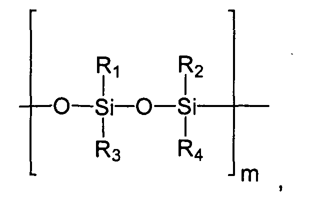 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt 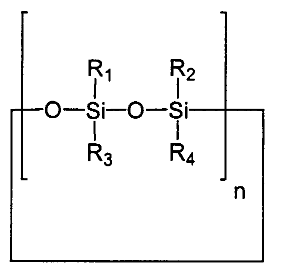 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan), Cetyldimethicon, Behenoxydimethicon.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-lsostearat (und) Hexyllaurat)

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate.

Erfindungsgemäße als Emulsionen vorliegenden Zubereitungen enthalten insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Erfindungsgemäße als Hydrogele vorliegenden Zubereitungen enthalten ein oder mehrere Hydrocolloide. Diese Hydrocolloide können vorteilhaft aus der vorgenannten Gruppe gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Vorteilhaft sind außerdem Taurate, z.B. Ammonium Acryloyldimethyltaurat / VP Copolymer.

Unter den Polyacrylaten befinden sich z.B. Carbopol Typen der Firma Goodrich (Carbopol 980, 981, 1382, 5984, 2984, ETD 2001, ETD 2020, ETD 2050, Ultrez-10 oder Pemulen TR1 & TR2).

Unter den Polymeren befinden sich z.B. Polyacrylamide (Seppigel 305), Polyvinylalkohole, PVP, PVP / VA Copolymere, Polyglycole.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße als Emulsionen vorliegenden Zubereitungen enthalten einen oder mehrere Emulgatoren. Diese Emulgatoren können vorteilhaft gewählt werden aus der Gruppe der nichtionischen, anionischen, kationischen oder amphoteren Emulgatoren.

Unter den nichtionischen Emulgatoren befinden sich
a) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)
b) ethoxylierte Fettalkohole und Fettsäuren
c) ethoxylierte Fettamine, Fettsäureamide, Fettsäurealkanolamide
d) Alkylphenolpolyglycolether (z.B. Triton X)
e) Zuckerderivate (Ester und/oder Ether von Glucose, Saccharose und anderen Zuckern; z.B. Alkylpolyglycoside wie Polyglyceryl-3-Methylglucose-Distearat, Methylglucosesesquistearat)

Unter den anionischen Emulgatoren befinden sich
a) Seifen (z. B. Natriumstearat)
b) Fettalkoholsulfate
c) Mono-, Di- und Trialkylphosphosäureester und deren Ethoxylate

Unter den kationischen Emulgatoren befinden sich
a) quaternäre Ammoniumverbindungen mit einem langkettigen aliphatischen Rest z.B. Distearyldimonium Chloride

Unter den amphoteren Emulgatoren befinden sich
a) Alkylamininoalkancarbonsäuren
b) Betaine, Sulfobetaine
c) Imidazolinderivate

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH nd

   n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel

   R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19-), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)-cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß ist auch die Verwendung partikulärer organischer Lichtschutzfilter zum Schutz vor farblichen Veränderungen kosmetischer und/oder dermatologischer Zubereitungen enthaltend wasserlösliche Wirkstoffe.

Die erfindungsgemäßen kosmetischen und/oder dermatologischen Zubereitungen finden erfindungsgemäß vorteilhaft Verwendung
- zum Schutz vor Hautalterung
- als Radikalfänger
- zur Bindung von schädlichen Photoprodukten an Lipide, DNS und Proteine
- zur Förderung Zellerneuerung und Regeneration der Haut
- zur Behandlung und Prophylaxe von Pigmentierungsstörungen, trockenen Hautzuständen und Hornschicht-Barrierestörungen
- zur Behandlung und Prophylaxe von Falten, Altersflecken, Teleangiektasien
- zur Behandlung und Prophylaxe von schädlichen Photoreaktionen der Haut und lichtbedingten Hautschäden.

Besonders vorteilhaft ist die Verwendung der erfindungsgemäßen kosmetischen und/oder dermatologischen Zubereitungen zur Bekämpfung und/oder Prophylaxe des oxidativen Stresses sowie lichtbedingter Hautalterungseffekte, insbesondere von Hautfältchen.

Die erfindungsgemäßen kosmetischen und/oder dermatologischen Zubereitungen können beispielsweise erfindungsgemäß vorteilhaft verwendet als Crèmes, Lotionen, kosmetische Milchzubereitungen, Reinigungsemulsionen oder Mousse-Cremes, die aus einem Aerosolbehälter austragbar sind. Auch in wässrigen oder wässrig-ethanolischen Lösungen, z.B. als Tränkungsmedium für Tücher oder als sprühbare Lösung bzw. Emulsion, wasserfreien oder wasserhaltigen Stiftformulierungen und Mikroemulsionen sind die erfindungsgemäßen Zubereitungen erfindungsgemäß vorteilhaft einsetzbar.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele 1-10: O/W-Cremes

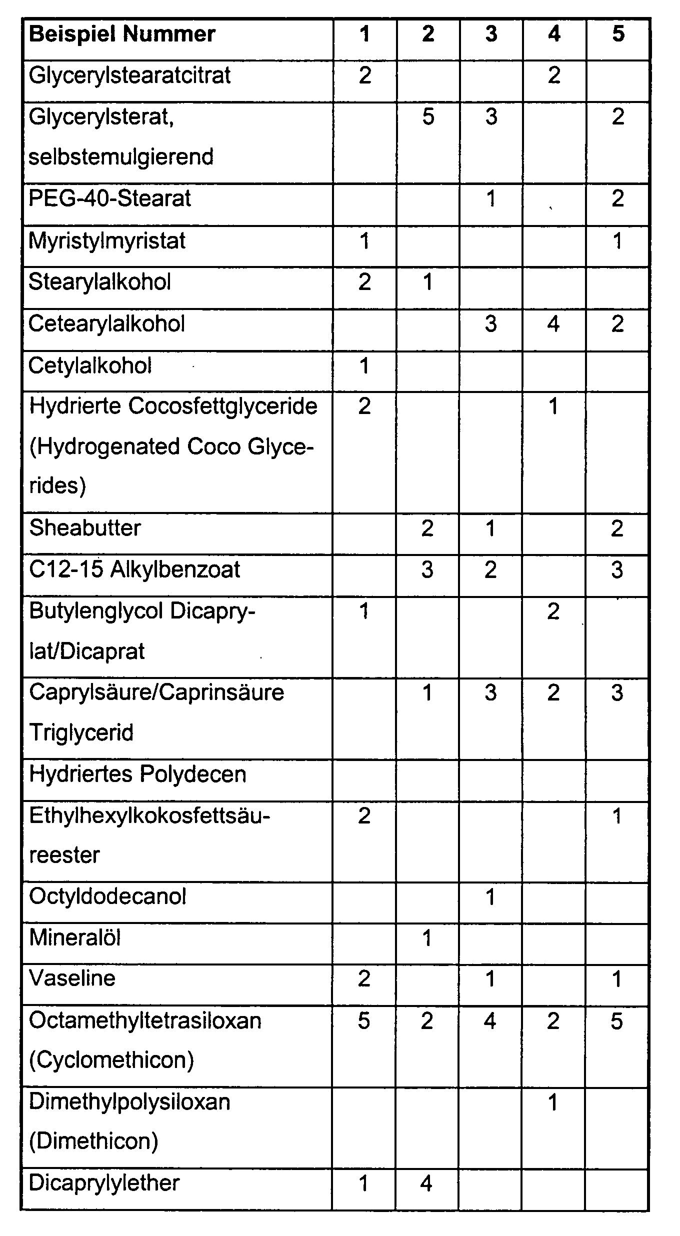

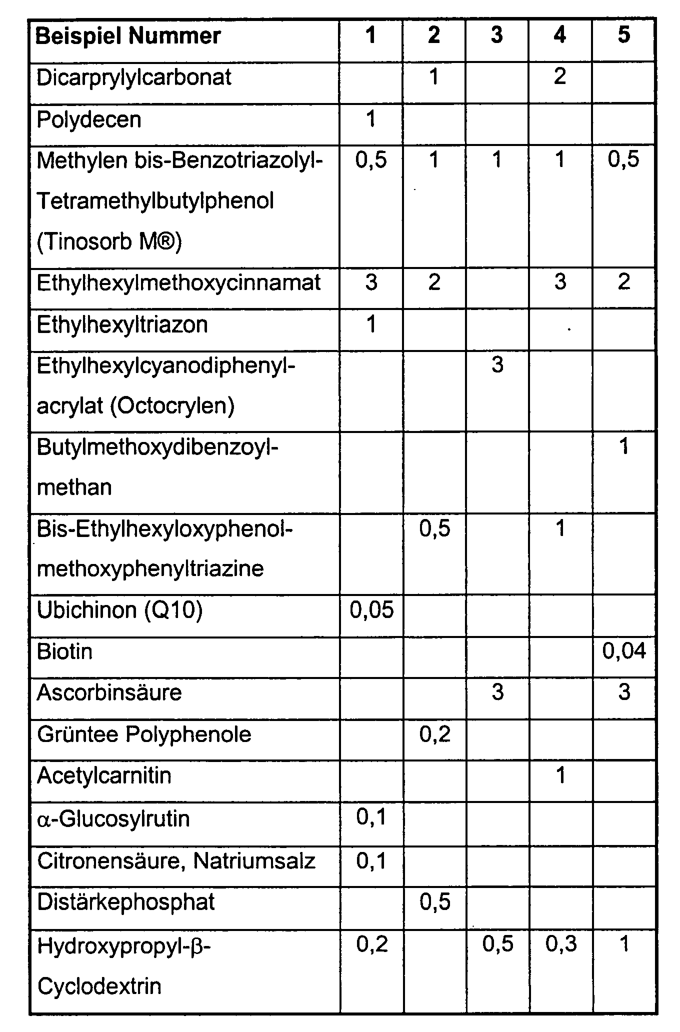

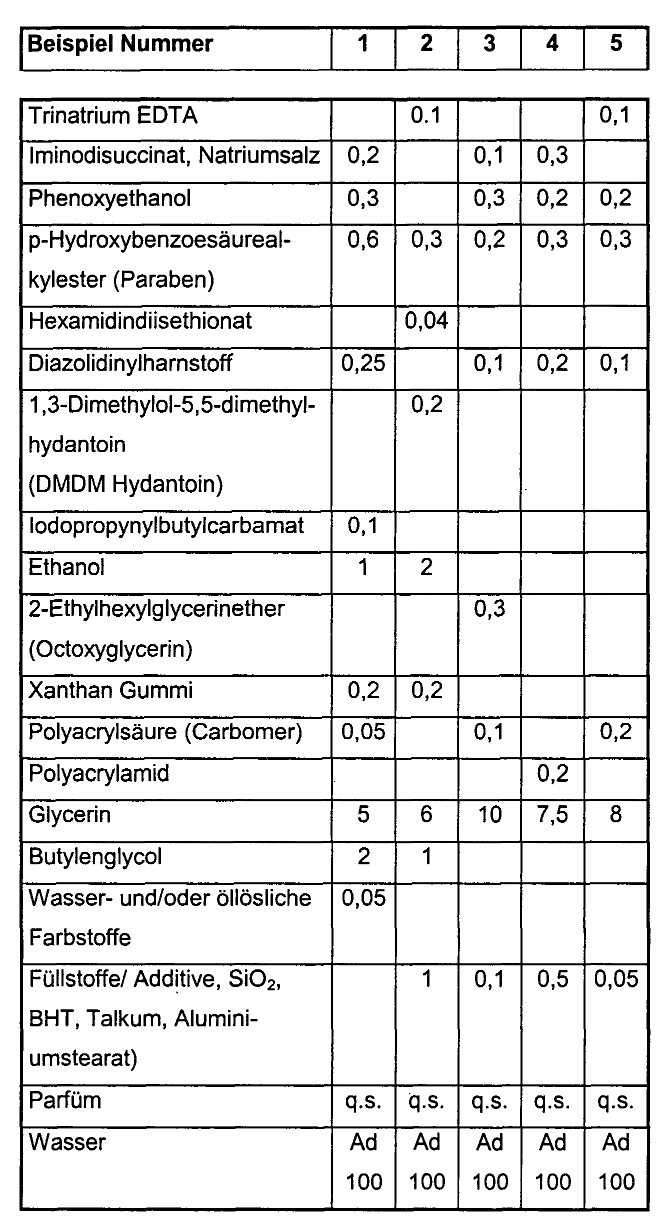

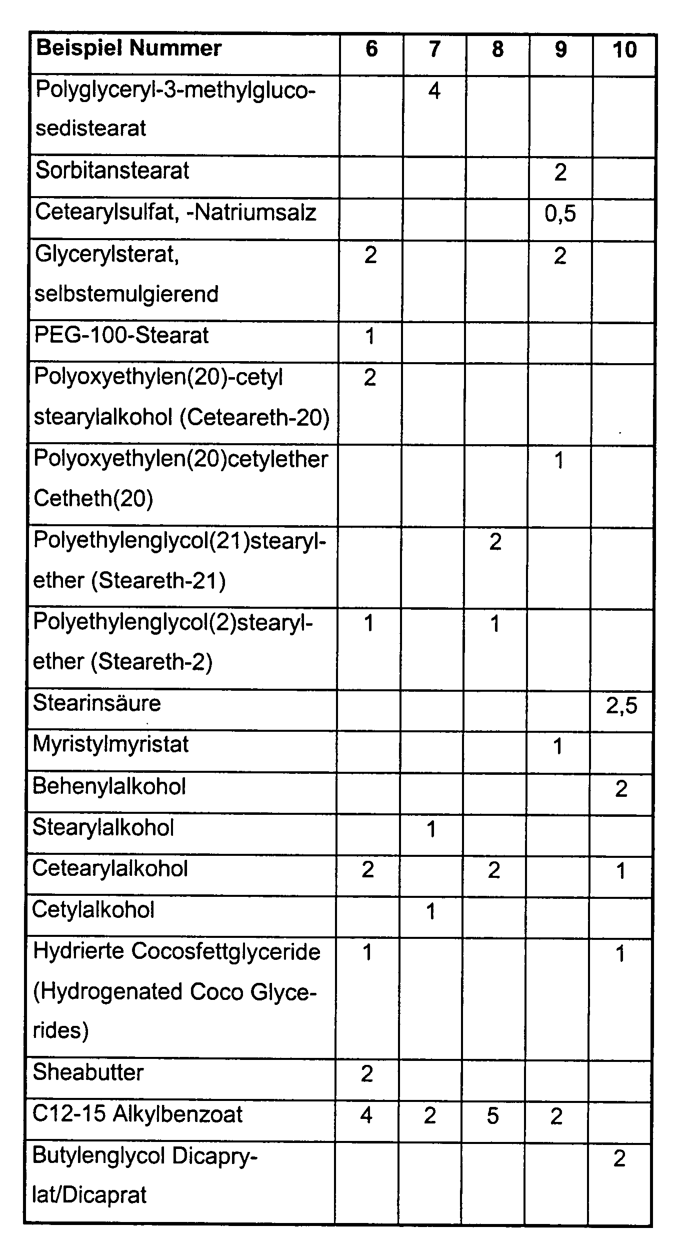

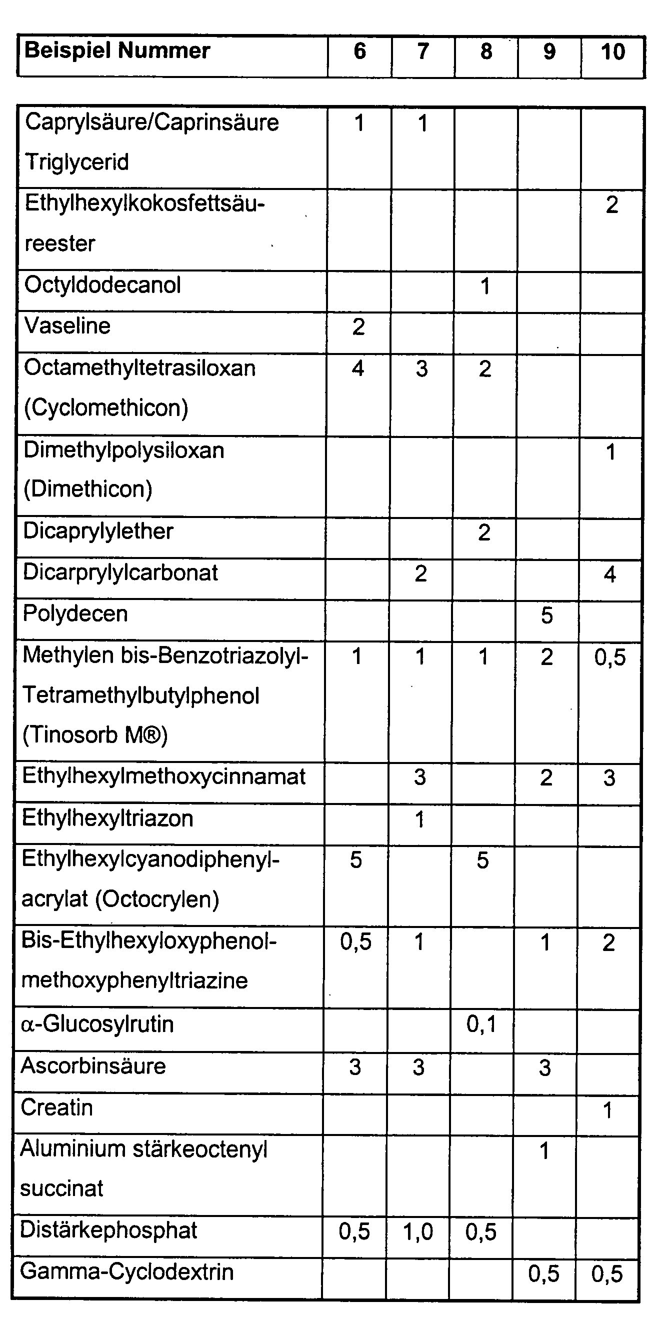

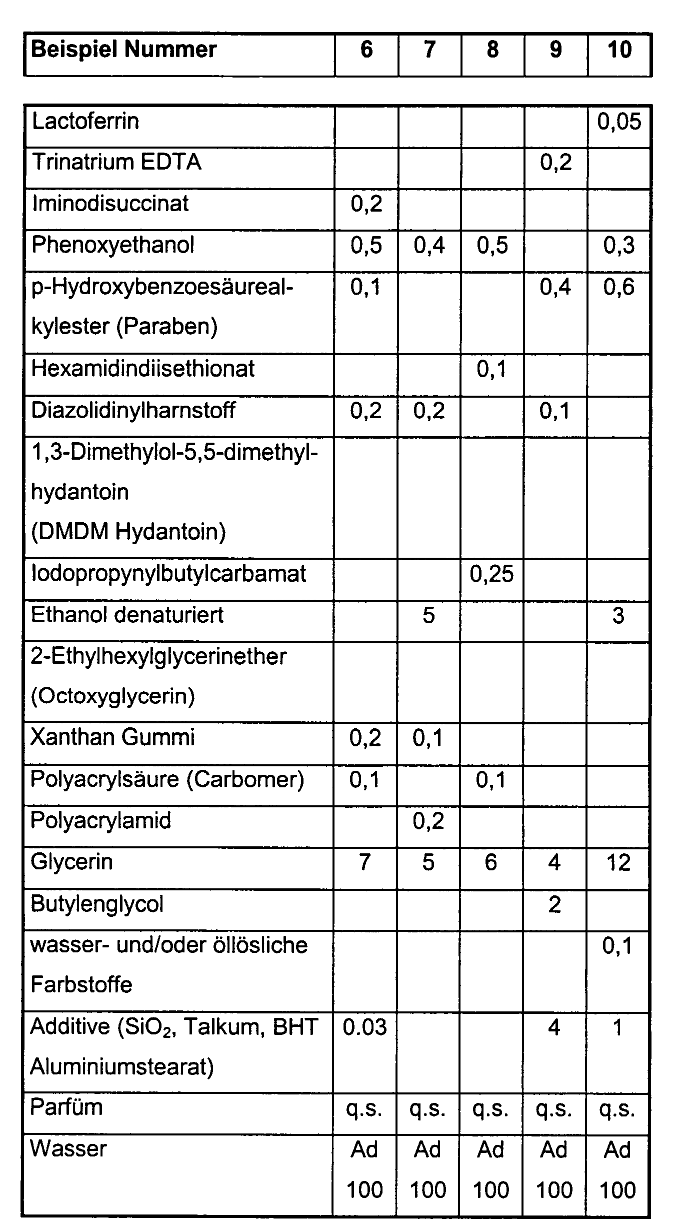

| **Beispiel 11: W/O-Creme** | |
|---|---|
| Polyglyceryl-3-Diisostearat | 5,0 |
| Polyglyceryl-2-Dipolyhydroxystearat | 2,5 |
| Cetearylalkohol | 2 |
| Cetylalkohol | 2 |
| C12-15 Alkylbenzoat | 8 |
| Caprylsäure/Caprinsäure Triglycerid | 6 |
| Octyldodecanol | 5 |
| Octamethyltetrasiloxan (Cyclomethicon) | 2 |
| Milchsäure | 5 |
| Citronensäure, Natriumsalz | 0,5 |
| Methylen bis-Benzotriazolyl-Tetramethylbutylphenol | 1 |
| Ethylhexyltriazon | 1 |
| Ethylhexylmethoxycinnamat | 5 |
| Ubichinon (Q10) | 0,05 |
| Ascorbinsäure | 0,5 |
| Phenoxyethanol | 0,1 |
| p-Hydroxybenzoesäurealkyleste (Paraben) | 0,1 |
| Glycerin | 7.5 |
| Distärkephosphat | 0.3 |
| Füllstoffe (SiO₂,Talkum, Aluminiumstearat) | 0,2 |
| Parfüm | q.s. |
| Wasser | Ad 100 |

| **Beispiel 12: Hydrodispersion/Gelcreme** | |
|---|---|
| Cetearylalkohol | 2 |
| Sheabutter | 1 |
| Caprylsäure/Caprinsäure Triglycerid | 2 |
| Octyldodecanol | 1 |
| Octamethyltetrasiloxan (Cyclomethicon) | 5 |
| Dimethylpolysiloxan (Dimethicon) | 1 |
| Polydecen | 2 |
| Methylen bis-Benzotriazolyl-Tetramethylbutylphenol (Tinosorb M®) | 0,4 |
| Ethylhexylmethoxycinnamat | 5 |
| Bis-Ethylhexyloxyphenol-methoxyphenyltriazin | 1 |
| Ascorbinsäure | 0,2 |
| Biotin | 0.1 |
| Hydroxypropyl-ß-Cyclodextrin | 0,3 |
| Iminodisuccinat | 0,2 |
| Phenoxyethanol | 0,3 |
| p-Hydroxybenzoesäurealkyleste (Paraben) | 0,4 |
| Vernetztes Alkylacrylat (Alkylacrylate Cosspolymer) | 0,2 |
| Glycerin | 5 |
| Parfüm | q.s. |
| Wasser | Ad 100 |

| **Beispielrezepturen Foundations** | | | | |
|---|---|---|---|---|
| **Beispiel Nummer** | **13** | **14** | **15** | **16** |
| PEG-30-stearat | 1,5 | | | |
| Gylcerylstearat | 0,5 | | | 2 |
| Ceteareth-20 | 1 | | | |
| Polyglyceryl-3 Methylglucosestearat | | | 3,5 | |
| Sorbinsäurestearat | | | 1,5 | |
| Stearinsäure | 2 | | | 1,8 |
| Glycerylstearatcitrat | | 3,5 | | |
| Cetylalkohol | 0,5 | 0,75 | 1,0 | |
| Lecithin | | | | 0,5 |
| Veegum K = Mg-AI-Silicate | 0,8 | | | 1 |
| Xanthan Gum | 0,2 | | | 0,3 |
| Carbomer | | 0,2 | | |
| Hydroxyethylcellulose | 0,2 | | 0,1 | |
| Caprylsäure / Caprinsäure Triglycerid | | 2 | 3 | 2 |
| Dicaprylylether | | 2 | 3 | 3 |
| Octyldodecanol | | | 3 | |
| Dimethicon | 3 | | 3 | 3 |
| Cyclomethicon | | 4 | 3 | 2 |
| C12-C15 Alkyl Benzoate | 2 | | | |
| Cetearyloctanoat | 2 | | | |
| Squalan | 1 | | | 6 |
| Isopropylpalmitat | 1 | | | 2 |
| PPG -15 Stearylether | 2 | 3 | | |
| Hydrierete Cocos-Glyceride | 2 | | | |
| Stearyldimethicon | 9 | | | |
| Acetyliertes Lanolinöl | 2 | 0,2 | | |
| Ethylhexylmethoxycinnamat | 3 | 2 | | 2 |
| Methylen bis-Benzotriazolyl-Tetramethylbutylphenol | 0,5 | 0,4 | 1 | 0,5 |
| Ethylhexyltriazon | 1 | | | 1 |
| Bis-Ethylhexyloxyphenolmethoxyphenyltriazin | 0,5 | | 1 | |
| Ascorbinsäure | 0,5 | 0,1 | 0,3 | 0,8 |
| Biotin | 0.01 | 0.025 | 0.1 | 0.05 |
| Silikon | | 0,8 | | |
| Nylon-12 | | | 5 | |
| Lauroyllysin | 0,5 | | 0,5 | |
| Kaolin | 0,5 | | 1 | 2 |
| Natrium Stärke Octenylsuccinat | | 1 | | 1,5 |
| Eisenoxide | 1,2 | 2,4 | 2,6 | 3 |
| Interferenz Pigmente | 0,2 | | | 0,5 |
| Pigment Low Lustre | 0,2 | | | 0,2 |
| Polymethylsilsesquioxan (Tospearl 2000B) | | 2 | | |
| Distärkephosphat | 0,3 | 0,2 | 1 | 0,5 |
| EDTA | 0,1 | 0,6 | 1 | 1 |
| Glycerin | 2 | 5 | 5 | 10 |
| Phenoxyethanol und Paraben (Phenonip) | 1 | 0,5 | | 1 |
| Imidazonidinylharnstoff | 0,3 | 0,3 | | 0,25 |
| Neutralisationsmittel | q.s. | q.s. | q.s. | q.s. |
| Parfum, Antioxidantien | q.s. | q.s. | q.s. | q.s. |
| Wasser | ad 100 | ad 100 | ad 100 | ad 100 |

| **Wasserhaltige Stifte** | | | | |
|---|---|---|---|---|
| **INCI** | **1** | **2** | **3** | **4** |
| Ricinus Communis | ad.100 | | | |
| Caprylic/Capric Triglyceride | 20,0 | ad 100 | 5,0 | 5,0 |
| Octyldodecanol | 20,0 | 7,0 | 5,0 | 5,0 |
| Paraffin Oil | | 5,0 | | |
| Pentaerythrityl Tetraisostearate | | 2,0 | 4,0 | |
| Carpylyl Carbonate | | | | 5,0 |
| Buxus Chinensis | 6,0 | 2,0 | 1,0 | 1,0 |
| Lanolin Oil | 5,0 | | | |
| Simethicone | | | 0,5 | 0,5 |
| PEG-45/ Dodecyl Glycol Copolymer | | | 3,5 | 2,0 |
| Polyglyceryl-3 Diisostearate | 3,7 | 2,5 | | 1,5 |
| Bis-Diglyceryl Polyacyladipate-2 | | 9,0 | 2,0 | |
| Cetearyl Alcohol | 6,0 | 10,0 | | |
| Cetyl Palmitate | 7,5 | 3,5 | | |
| C16-36 Alkyl Stearate | | 17,0 | 1,0 | 2,0 |
| C18-38 Alkyl Hydroxystearoyl Stearate | | 2,0 | | |
| C20-40 Alkyl Stearate | 16,0 | | 8,0 | 8,0 |
| Cera Carnauba | | 1,5 | 1,5 | 0,5 |
| Cera Alba | 6,0 | 0,5 | | |
| PVP / Eicosense Copolymer | | 1,0 | | |
| Methylen bis-Benzotriazolyl-Tetramethylbutylphenol | 0,5 | 1 | 0,3 | 1 |
| Butyl Methoxydibenzoylmethane | 1 | | 0,5 | |
| Octyl Methoxycinnamate | 3,0 | 3,0 | 2,0 | 2,0 |
| Nylon-12 | | | 3,0 | |
| BiOCl₃ | | | | 3,0 |
| Lauroyl Lysine | 0,5 | | 0,5 | |
| Polymethylsilsesquioxane | | | | 0,5 |
| PMMA | | | 6,0 | 3,0 |
| Diammonium Citrate | 0,08 | | | |
| Ascorbinsäure | | 0,2 | 0,5 | 1 |
| Grüntee Flavonoide | 0,2 | | | |
| Zitronensäure | 0,05 | | | 0,05 |
| Glycerin | | | | 10 |
| Panthenol | | 0,5 | | |
| Distärkephosphat | 0,5 | 0,2 | 1 | 0,3 |
| Parfum, Konservierungsmittel, BHT, Neutralisationsmittel, Sequestriemittel | q.s | q.s | q.s | q.s |
| Aqua | 2,5 | 5,0 | ad 100 | ad 100 |

## Patentansprüche

1. Kosmetische und/oder dermatologische Zubereitungen enthaltend mindestens einen wasserlöslichen Wirkstoff und mindestens einen partikulären organischen UV-Lichtschutzfilter.

2. Kosmetische und/oder dermatologische Zubereitungen nach Anspruch 1, **dadurch gekennzeichnet, dass** die wasserlöslichen Wirkstoffe in einer Konzentration von 0,001 bis Gewichts- 10,0% bezogen auf das Gesamtgewicht der Zubereitung eingesetzt werden.

3. Kosmetische und/oder dermatologische Zubereitungen nach einem der Ansprüche 1 oder 2, **dadurch gekennzeichnet, dass** die partikulären organischen Lichtschutzfilter in einer Konzentration von 0,01 bis 10 Gewichts-% bezogen auf das Gesamtgewicht der Zubereitung eingesetzt werden.

4. Kosmetische und/oder dermatologische Zubereitungen nach einem der Ansprüche 1 bis 3, **dadurch gekennzeichnet, dass** das Verhältnis von wasserlöslichen Wirkstoffen zu partikulären organischen Lichtschutzfiltern von 1 : 500 bis 50 : 1 beträgt.

5. Kosmetische und/oder dermatologische Zubereitungen nach einem der Ansprüche 1 bis 4, **dadurch gekennzeichnet, dass** als wasserlösliche Wirkstoffe Ascorbinsäure, α-Glucosylrutin, Carnitin und/oder dessen Derivate, Flavonoide des grünen Tees und/oder Creatin und/oder dessen Derivate eingesetzt werden.

6. Kosmetische und/oder dermatologische Zubereitungen nach einem der Ansprüche 1 bis 5, **dadurch gekennzeichnet, dass** als partikulärer organischer Lichtschutzfilter 2,2'-Methylen-bis-(6-(2H-benzotriazol-2-yl)-4-(1,1,3,3-tetramethylbutyl)-phenol) eingesetzt wird.

7. Kosmetische und/oder dermatologische Zubereitungen nach einem der Ansprüche 1 bis 6, **dadurch gekennzeichnet, dass** sie lineare, verzweigte und/oder cyclische Oligo- und/oder Polysaccharidderivate enthalten.

8. Kosmetische und/oder dermatologische Zubereitungen nach einem der Ansprüche 1 bis 7, **dadurch gekennzeichnet, dass** die linearen, verzweigten und/oder cyclischen Oligo- und/oder Polysaccharidderivate aus der Gruppe der Cyclodextrine, Distärkephosphat, nichtgelierende Cellulosen, Natriumstärkeoctenylsuccinat und Aluminiumstärkeoctenylsuccinat gewählt werden.

9. Kosmetische und/oder dermatologische Zubereitungen nach einem der Ansprüche 1 bis 8, **dadurch gekennzeichnet, dass** die linearen, verzweigten und/oder cyclischen Oligo- und/oder Polysaccharidderivate in einer Konzentration von 0,001 bis 10 Gewichts-%, bezogen auf das Gesamtgewicht der Zubereitung eingesetzt werden.

10. Verwendung partikulärer organischer Lichtschutzfilter nach einem der vorhergehenden Ansprüche zum Schutz vor farblichen Veränderungen kosmetischer und/oder dermatologischer Zubereitungen enthaltend wasserlösliche Wirkstoffe.

11. Verwendung von kosmetischen und/oder dermatologischen Zubereitungen nach einem der vorhergehenden Ansprüche als Creme, Lotion, Spray, Mousse, wässrige oder wässrig-ethanolische Lösung, Tränkungsmedium für Tücher, wasserfreier oder wasserhaltiger Stift, Mikroemulsion.
